# EUROPÄISCHE PATENTANMELDUNG

(11) **EP 2 966 581 A1**
(43) Veröffentlichungstag der Anmeldung: **13.01.2016**
(21) Anmeldenummer: 14175985.2
(22) Anmeldetag: 07.07.2014
(51) Int. Cl.: G06F 19/00

(54) **Netzwerksystem und Verfahren zur Steuerung eines Computertomographen**

(71) Anmelder: Siemens Aktiengesellschaft, 80333 München (DE)
(72) Erfinder: Seifert, Sascha, 91052 Erlangen (DE)

(57) **Zusammenfassung**

Die Erfindung betrifft ein Computer-implementiertes Netzwerksystem und ein Verfahren zum Zugriff auf eine Dosis-Bild-Datenbank (DDB) für die medizinische Bildgebung unter Anwendung von ionisierender Strahlung mittels einer bildgebenden Einrichtung (10). Das Netzwerk umfasst eine Vielzahl von bildgebenden Einrichtungen (10), die über eine Netzwerkverbindung (nw) an die Dosis-Bild-Datenbank (DDB) angeschlossen sind, wobei jede bildgebende Einrichtung (10) eine Sendeeinheit (Se) umfasst, die dazu bestimmt ist, die von der bildgebenden Einrichtung (10) erfassten Bilder an die Dosis-Bild-Datenbank (DB) zu senden. In der Dosis-Bild-Datenbank (DDB) ist eine Zuordnung zwischen einem Bild oder eine Klasse von Bildern, einem Dosismesswert, einer Bildqualitätsmesswert und optional noch weiteren medizinischen Metadaten abgelegt. Das System weist ebenso auf eine Steuereinheit (S), die in Datenaustausch steht mit der Dosis-Bild-Datenbank (DDB) und die dazu dient, unter Zugriff auf die Dosis-Bild-Datenbank (DDB) automatisch Steuergrößen in Abhängigkeit von zumindest einem zu bestimmenden Auswahlparameter zu erzeugen, die über eine Netzwerkverbindung (nw) an zumindest eine bildgebende Einrichtung (10) zur Steuerung der Bildgebung weitergeleitet werden.

## Beschreibung

### GEBIET DER ERFINDUNG

Die vorliegende Erfindung liegt auf den Gebieten der Medizintechnik und der Informationstechnologie bzw. der elektronischen Steuerungen und betrifft insbesondere die Steuerung von bildgebenden Einrichtungen, die zur Bildgebung ionisierende Strahlung einsetzen.

### STAND DER TECHNIK

Bildgebende medizinische Einrichtungen verwenden ionisierende Strahlung, um auswertbare Bilddaten zu erzeugen, wie z.B. Computertomographen oder Fluoroskopiegeräte. Ein grundsätzliches Ziel ist es, den Patienten möglichst nur einer solchen Strahlendosis auszusetzen, die unbedingt erforderlich ist, um eine ausreichende Bildqualität gewährleisten zu können. Dabei stehen die zu applizierende Strahlendosis und die Bildqualität in einem konkurrierenden Verhältnis, so dass die Strahlendosis jeweils durch Abwägung der beiden Aspekte zu bestimmen ist, was eine automatische Steuerung der Strahlendosis erschwert. Die Dosisermittlung ist ein wichtiger Vorbereitungsschritt bei der Planung, Durchführung und/oder Steuerung des Gerätes bei einem strahlentherapeutischen oder nuklearmedizinischen Vorgang.

Neben der Abwägung, wieviel an Dosiseinsparung erzielbar ist, ohne störende Einbußen in der Bildqualität hinnehmen zu müssen, steht der Anwender einer Vielzahl von Untersuchungsparametern gegenüber. Dosismessungen sind modalitäten-spezifische Mess- oder Schätzverfahren (z.B. für CT: volume CT dose index (CTDIvol), and dose length product (DLP); für die Fluoroskopie: dose area product (DAP), kerma area product (KAP), cumulative air kerma (CAK), and entrance surface dose (ESD) etc.) Die Auswirkung von Änderungen einzelner oder mehrerer dieser Parameter auf die Bildqualität und Dosis ist oft nicht ohne weiteres durch den Anwender einschätzbar. Zudem gibt es hersteller-spezifische Parameter, deren technischer Hintergrund dem Anwender oft nicht im Detail bekannt ist. Die Optimierung der Dosis ist noch dazu abhängig vom Typ der bildgebenden Untersuchung (eingesetzte Modalität und Art der Untersuchung, wie z.B. Thorax CT und Abdomen CT).

Oft setzen Radiologen bei Verfahren gemäß dem Stand der Technik angesichts dieser Komplexität auf Heuristiken und Erfahrungswerte in der Erstellung von Untersuchungsprotokollen. Die dabei verwendbaren Qualitätssicherungsmaßnahmen sind jedoch leider sehr beschränkt. So ist es zwar möglich, institutionsbezogene Qualitätssicherungsmaßnahmen zu ergreifen, indem z.B. Radiologen institutionsbezogene Standards für Untersuchungsprotokolle festlegten, die sich an publizierten Studien führender Zentren orientieren. All diese Maßnahmen setzen entweder allgemeine (und nicht fallspezifische) Heuristiken ein, konzentrieren sich auf bestimmte Untersuchungstypen oder die Beurteilung der Dosis(verteilung) anhand von phantom-basierten Messungen (modell-basierte Dosis-Messungen unter kontrollierten Bedingungen, die nicht die Patientenkonstitution und nur eingeschränkt Details anatomischer Strukturen berücksichtigen).

Eine patienten-orientierte und patienten- und fall-spezifische Vorhersage und Optimierung von Untersuchungsparametern zur Dosisoptimierung ist auf dieser Basis nur eingeschränkt oder gar nicht möglich. Die Heuristiken lassen eine Beeinflussung der Dosis innerhalb bestimmter Größenordnungen zu, d.h. es werden oft Intervalle für Untersuchungsparameter angegeben oder fixe Protokolle, die die individuelle Patientenkonstitution, die verwendete Hard- und Software sowie Besonderheiten des Untersuchungsablaufs gar nicht oder nur unzureichend berücksichtigen. Oft besteht ein erster Schritt in der Qualitätssicherung in der Vermeidung von Ausreißern, d.h. von Untersuchungen bei denen eine offensichtlich zu hohe Dosis verwendet wird und nicht in einer feingranularen Dosisoptimierung. Wegen der hohen Komplexität der relevanten Parameter kann für viele Untersuchungsvarianten nicht ohne weiteres auf Publikationen zurückgegriffen werden, die speziell angepasste Lösungen für die aktuelle Problemstellung (komplexe Konstellation aus Untersuchungstyp, Fragestellung, Patientenkonstitution und Gerätetyp) beschreiben.

Ausgehend von diesem Stand der Technik hat sich die vorliegende Erfindung zur Aufgabe gemacht, ein Netzwerksystem mit einer Dosis-Bild-Datenbank zum übergreifenden Austausch von dosis-relevanten Datensätzen für die medizinische Bildgebung mit ionisierender Strahlung bereitzustellen. Dabei soll es möglich sein, für eine anstehende Bildgebung, die für den jeweiligen Fall optimalen Steuerungsgrößen mit einer Berechnung einer Strahlendosis zu bestimmen. Für diese Berechnung soll die Bildqualität von einer Vielzahl von Voruntersuchungen ausgewertet werden können. Insgesamt soll die Strahlungsintensität für den Patienten bei einer bildgebenden Untersuchung verringert werden bei Einhaltung einer ausreichenden Bildqualität.

Diese Aufgabe wird durch die beiliegenden nebengeordneten Patentansprüche gelöst, also durch ein Verfahren, ein Produkt (insbesondere auch Computerprogrammprodukt) und ein Netzwerksystem.

Nachstehend wird die Lösung der Aufgabe in Bezug auf das beanspruchte Systen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die nicht-gegenständlichen Ansprüche (die beispielsweise auf ein Verfahren gerichtet sind) auch mit den Merkmalen, die in Zusammenhang mit dem System oder Netzwerk beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Gemäß einem Aspekt betrifft die Erfindung ein Netzwerksystem zum Bereitstellen einer Dosis-Bild-Datenbank für die medizinische Bildgebung unter Anwendung von ionisierender Strahlung mittels einer bildgebenden Einrichtung, mit:
- Einer Vielzahl von bildgebenden Einrichtungen, die über eine Netzwerkverbindung an die Dosis-Bild-Datenbank angeschlossen sind, wobei jede bildgebende Einrichtung eine Sendeeinheit umfasst, die dazu bestimmt ist, die alle oder ausgewählte von der bildgebenden Einrichtung erfassten Bilder an die Dosis-Bild-Datenbank zu senden, wobei in der Dosis-Bild-Datenbank eine Zuordnung zwischen einem Bild oder eine Klasse von Bildern, einem Dosismesswert, einer Bildqualitätsmesswert und optional noch weiteren medizinischen Metadaten abgelegt ist
- Einer Steuereinheit, die in Datenaustausch steht mit der Dosis-Bild-Datenbank und die dazu dient, unter Zugriff auf die Dosis-Bild-Datenbank automatisch Steuergrößen in Abhängigkeit von zumindest einem zu bestimmenden Auswahlparameter zu erzeugen, die über eine Netzwerkverbindung an zumindest eine bildgebende Einrichtung zur Steuerung der Bildgebung weitergeleitet werden.

Im Folgenden werden die im Rahmen dieser Anmeldung verwendeten Begrifflichkeiten näher erläutert.

Das Netzwerksystem ist computer-basiert. Es umfasst in der bevorzugten Ausführungsform mehrere Recheneinheiten, Prozessoren, Computer und/oder Netzwerke. Es kann ein lokales Netzwerk sein (LAN: local area network) und/oder ein Weitverkehrsnetzwerk (WAN: wide area network) mit entsprechenden Zwischensystemen, wie Switches, Paketvermittlern, Bridges und Routern. Das LAN kann institutsintern oder institutsübergreifend betrieben und implementiert sein, um mehrere unterschiedliche klinische Bereiche zu verbinden. Dementsprechend kann es sich gemäß einer Ausführungsform bei der Netzwerkverbindung um eine LAN-Verbindung oder gemäß einer alternativen Ausführungsform um eine WAN-Verbindung handeln. Letztere kann auch über eine Internetverbindung oder eine Mobilfunkverbindung implementiert sein.

Das Netzwerksystem dient zum Bereitstellen der Dosis-Bild-Datenbank. Darüber hinaus kann es auch zum Zugriff auf die Dosis-Bild-Datenbank eingesetzt werden. Der Zugriff kann ein Schreib (WRITE-), eine Lese (READ-) oder eine Ausführzugriff (EXE) sein.

Bei der bildgebenden Einrichtung handelt es sich um ein medizintechnisches Gerät zur Bildakquisition. In einer bevorzugten Ausführungsform erfolgt die Bildakquisition unter Anwendung von ionisierender Strahlung, wie z.B. mittels eines Computertomographen, Computer Radiografiegerätes, Röntgengerätes, Tomosynthesegerätes und/oder Geräten zur Fluoroskopie. Im weiteren Sinne können auch andere Geräte mit dem erfindungsgemäßen Verfahren gesteuert werden, die eine Dosisbestimmung erfordern.

Steuergrößen (auch Steuerparameter genannt) sind Parameter, die bei einer Anwendung des medizinischen Gerätes eingestellt werden müssen. Gemäß einem Aspekt sind Strahlendosisdaten auch von den Steuergrößen umfasst. Des Weiteren zählen dazu, die Bestimmung eines Rekonstruktionsalgorithmus, den Tischvorschub oder weitere technische Geräteparameter. Die Steuergrößen sind die Einstellungen (settings), die bei einer zukünftigen bzw. geplanten Anwendung der ionisierenden Strahlung verwendet und deren Dosis ermittelt und zur Steuerung des Gerätes verwendet werden sollen.

Die Steuereinheit dient zur Berechnung der Steuergrößen, die von ihr an die jeweiligen bildgebenden Einrichtungen weitergeleitet werden. Die Steuereinheit kann auch als Dosisbestimmungseinheit ausgebildet sein und somit eine eingeschränkte Funktionalität haben, nämlich, um die Dosis für eine anstehende Untersuchung unter Rückgriff auf eine Berechnung auf Basis von einer Vielzahl von Voraufnahmen und deren Auswertung hinsichtlich der Bildqualität zu bestimmen. Weiter kann die Steuereinheit auch automatisch ein Protokoll für die bildgebende Einrichtung und die anstehende Untersuchung bestimmen und, gegebenenfalls nach Empfang eines Bestätigungssignals hin, automatisch anwenden. Die Steuereinheit kann weiter dazu ausgebildet sein, zusätzlich eine Visualisierungseinheit zu umfassen oder auf diese zuzugreifen, wobei die Visualisierungseinheit dazu bestimmt ist, von der Steuereinheit berücksichtige Bilder bzw. Referenzbilder zu visualisieren. Dies bietet den Vorteil, dass ein Anwender unmittelbar einsehen kann, welche Bilder als Referenzbilder zu Dosisbestimmung und zur Berechnung der Steuergrößen verwendet worden sind.

Die Steuereinheit kann in einer Ausführungsform unmittelbar in der Dosis-Bild-Datenbank angeordnet sein oder als separate computer-basierte Instanz zwar an die Dosis-Bild-Datenbank angeschlossen, aber nicht in dieser implementiert und zentral für alle bildgebenden Einrichtungen bereitgestellt werden (z.B. in einer Cloud oder in einem Rechencenter). In einer alternativen Ausführungsform kann die Steuereinheit auch lokal und verteilt ausgebildet und auf allen oder ausgewählten bildgebenden Einrichtungen bereitgestellt werden. Es ist auch möglich, ausgewählte bildgebende Einrichtungen zu bestimmen, auf denen die Steuereinheit eingebaut ist und die dann für die anderen bildgebenden Einrichtungen sozusagen als Sendeinstanz zum Senden von Steuerparametern dient.

In der Dosis-Bild-Datenbank sind alle dosis-relevanten Informationen abgelegt. Die Informationen werden laufend (kontinuierlich oder gemäß einer bevorzugten Ausführungsform nach vorbestimmten Kriterien (zeit- oder ereignisbasiert) aktualisiert. In der Dosis-Bild-Datenbank ist eine Zuordnung zwischen einem Bild oder eine Klasse von Bildern, einem Dosismesswert, einer Bildqualitätsmesswert und optional noch wieteren medizinischen Metadaten abgelegt. Bei der Klasse von Bildern können alle Bilder eine Klasse oder Gruppe bilden, die für die Bestimmung der Dosis vergleichbare Werte haben. So können z.B. Bilder einer Studie hinsichtlich ihrer Qualität zusammengefasst werden und es muss die Zuordnungsrelation nur für einen Bildrepräsentanten der Gruppe speicherplatzsparend hinterlegt werden. Dabei kann automatisch ein Verweis hinterlegt sein, dass dieselbe Zuordnung auch für die anderen Bilder aus derselben Klasse gilt.

Wie oben bereits erwähnt, umfasst eine bildgebende Einrichtung üblicherweise eine Sendeeinheit. Die Sendeeinheit umfasst ist dazu bestimmt, alle oder ausgewählte von der bildgebenden Einrichtung erfassten Bilder an die Dosis-Bild-Datenbank zu senden. Ebenso ist es möglich, dass nicht - wie in der bevorzugten Ausführungsform vorgesehen - alle bildgebenden Einrichtungen ihre Bilder an die Dosis-Bild-Datenbank senden, sondern auch hier wieder bestimmte bildgebende Einrichtungen auszuwählen (insbesondere mit guter Netzwerkanbindung und/oder ausreichend Rechenleistung, dies kann somit auch dynamisch veränderlich bestimmt werden), die als Sender für die Bilder an die zentrale Dosis-Bild-Datenbank von anderen Modalitäten fungieren können.

Strahlendosisdaten ist die Gesamtmenge von Strahlendaten, die bei bisherigen und aktuellen Anwendungen von ionisierender Strahlung anfallen. Dabei wird die pro Zeiteinheit aufgenommene Strahlendosis als Dosisleistung (Einheit: Sv/s oder Sv/h) bezeichnet.

Gemäß einem Aspekt betrifft die Erfindung somit ein System zum Steuern einer bildgebenden Einrichtung über Steuergrößen, die die Bildqualität und die Strahlendosis in Kombination auswerten. Dies wird auf Basis der Auswertung von gespeicherten Bildern (oder Ausschnitten davon) ausgeführt, indem deren Bildqualität bei der jeweils verwendeten Strahlendosis und/ oder den verwendeten Akquisitionsparametern (Steuergrößen) verrechnet wird.

Ziel ist es, eine Dosiseinsparung zu erzielen bei Sicherstellung einer für die jeweilige Untersuchung ausreichenden Bildqualität. Die Bildqualität wird vorzugsweise anhand von automatisch ausgewerteten Referenzbildern als ausreichend oder nicht ausreichend analysiert. Erfindungsgemäß kann vorteilhafterweise die Menge der physikalischen Strahlendosis verringert werden, ohne dass eine relevante Einbuße in der Bildqualität hingenommen werden muss.

Gemäß einer bevorzugten Ausführungsform umfasst das Netzwerksystem eine Cluster-Einheit, die dazu bestimmt ist, für eine Menge von Bildern ein Referenzbild (als Bildrepräsentanten) zu bestimmen. Dies hat den Vorteil, dass in der Dosis-Bild-Datenbank dann nur für das Referenzbild die Zuordnungsrelation abgelegt werden muss. Es ist automatisch bestimmt, dass auch alle anderen Bilder der Klasse/des Clusters dieselbe Zuordnungsrelation gilt.

Die Auswahlparameter werden gebildet aus einer Menge von Parametern und können einen oder mehrere der folgenden Parameter umfassen:
- Patientenidentität
- Zu untersuchender, anatomischer Bereich,
- gewünschte Bildqualität
- weitere Metadaten, die gemäß einer bevorzugten Variante der Erfindung aus dem DICOM-Header (DICOM: Digital Imaging and Communications in Medicince) automatisch ausgelesen werden.

Das Netzwerksystem kann zusätzlich eine Qualitätsbestimmungseinheit umfassen, die dazu bestimmt ist, den Bildqualitätsmesswert automatisch zu bestimmen. Dazu können Bildverarbeitungsalgorithmen eingesetzt werden.

Die vorstehende Aufgabe wird ebenfalls gelöst durch ein computer-implementiertes Verfahren zum Austausch von Steuergrößen für eine bildgebende Einrichtung zur Bildgebung mit ionisierender Strahlung unter Zugriff auf eine Dosis-Bild-Datenbank, mit folgenden Verfahrensschritten:
- Automatisches Senden aller oder ausgewählter Bilder, die von einer oder mehreren bildgebenden Einrichtungen erfasst werden, über eine Netzwerkverbindung an eine Dosis-Bild-Datenbank,
- Erstellen einer Zuordnung und Speichern derselben in der Dosis-Bild-Datenbank zwischen einem Bild oder eine Klasse von Bildern, einem Dosismesswert, einer Bildqualitätsmesswert und optional noch weiteren medizinischen Metadaten
- Erzeugen von Steuergrößen in Abhängigkeit von zumindest einem zu bestimmenden Auswahlparameter unter Zugriff auf die Dosis-Bild-Datenbank,
- Weiterleiten der erzeugten Steuergrößen über eine Netzwerkverbindung an zumindest eine bildgebende Einrichtung zur Steuerung der Bildgebung.

Dabei umfasst das Verfahren zwei voneinander getrennte Abschnitte: Eine Bildsammelphase, in der die Bilder von den Modalitäten gesammelt ggf. verarbeitet und in der Dosis-Bild-Datenbank gespeichert werden und eine Steuerphase, in der unter Zugriff auf die Datenbank Steuergrößen abgeleitet und berechnet und für die Steuerung der Geräte verwendet werden. Beide Phasen sind voneinander unabhängig. Grundsätzlich wird die erste Phase der Bildsammlung vor der zweiten Steuerphase ausgeführt. Es liegt jedoch ebenso im Rahmen der Erfindung, die vorstehend erwähnten Schritte des Verfahrens nicht zwangsläufig in der vorstehend beschriebenen Reihenfolge zur Ausführung zu bringen. Alternativ ist es beispielsweise auch möglich, beide Phasen ineinander zu verschränken, so dass auch während der Steuerung zum Beispiel eines ersten Gerätes die Bilddaten von einem zweiten Gerät gesammelt und dem System zugeführt werden. So können die Verfahrensschritte des Einlesens der Bilddaten und des Erfassens von Steuergrößen ineinander verschachtelt sein (Interleaving), so dass bei Darstellung von Bilddaten unmittelbar ein Fenster auf dem Monitor des Befundungssystems aktivierbar ist, über das Steuerdaten im Hinblick auf die jeweils dargestellten Bilder eingegeben werden können. Dieses Fenster kann entweder für ein Bild oder für eine Gruppe von Bildern (z.B. eine Bildserie einer Untersuchung) - etwa durch einen Mausclick - aktiviert werden.)

Darüber hinaus ist es möglich, dass einzelne Abschnitte des vorstehend beschriebenen Verfahrens als einzelne verkaufsfähige Einheiten und restliche Abschnitte des Verfahrens als andere verkaufsfähige Einheiten ausgebildet werden können. Damit kann das erfindungsgemäße Verfahren als verteiltes System auf unterschiedlichen computer-basierten Instanzen (z.B. Client-Server-Instanzen) zur Ausführung kommen. So ist es beispielsweise möglich, dass die Steuereinheit seinerseits unterschiedliche Sub-Module umfasst, die teilweise auf der jeweiligen bildgebenden Einrichtung, teilweise auf der Dosis-Bild-Datenbank und/oder teilweise auf anderen computer-basierten Instanzen implementiert sind.

Eine weitere Lösung der vorstehenden Aufgabe bezieht sich auf ein Computerprogrammprodukt gemäß dem beiliegenden Anspruch. Eine weitere Aufgabenlösung sieht ein Computerprogramm vor, das Computerinstruktionen umfasst. Die Computerinstruktionen sind auf einem Speicher eines Computers gespeichert und umfassen von dem Computer lesbare Befehle, die zur Ausführung des vorstehend beschriebenen Verfahrens bestimmt sind, wenn die Befehle auf dem Computer ausgeführt werden. Das Computerprogramm kann auch auf einem Speichermedium gespeichert sein oder es kann über ein entsprechendes Netzwerk von einem Server heruntergeladen werden.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Figur 1: Eine schematische Darstellung von einem erfindungsgemäßen System mit einer Dosis-Bild-Datenbank und weiteren Instanzen gemäß einem Ausführungsbeispiel,
- Figur 2: ein Ablaufdiagramm eines Verfahrens gemäß einer bevorzugten Ausführungsform der Erfindung und
- Figur 3: eine Übersichtsdarstellung des Netzwerkes mit weiteren Einheiten und Instanzen gemäß einem Ausführungsbeispiel der Erfindung.

Im Folgenden wird die Erfindung anhand von Ausführungsbeispielen unter Bezugnahme auf die Figuren näher erläutert.

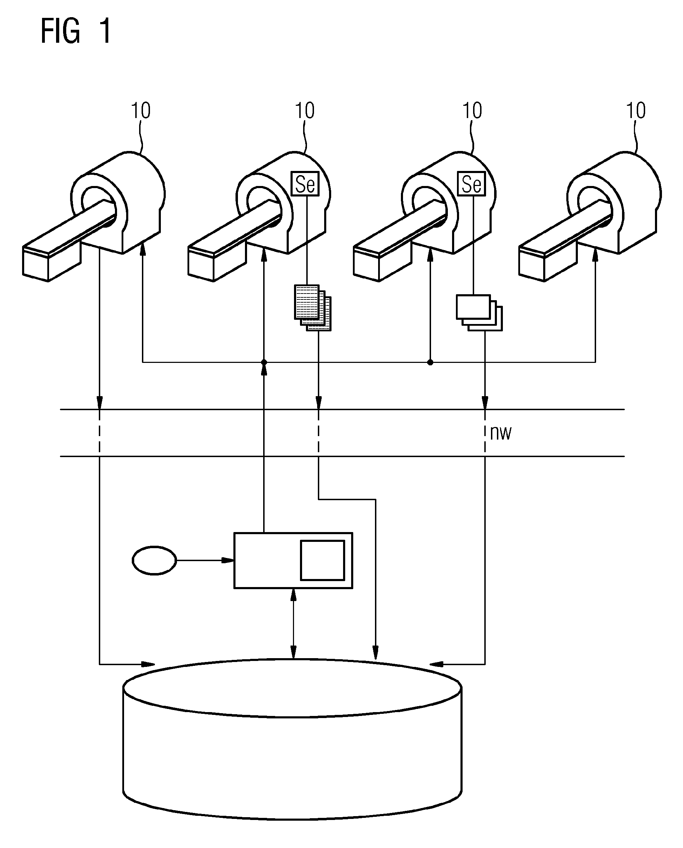

Die Erfindung betrifft ein Netzwerksystem, das schematisch in Figur 1 dargestellt ist. Mehrere bildgebende Einrichtungen (CT, klassisches Röntgen, MRT, PET, Ultraschall, etc.) oder auch Modalitäten 10 genannt können über ein oder mehrere Netzwerke nw in Datenaustausch untereinander und mit weiteren elektronischen Einheiten stehen. Das Netzwerksystem ist zum Crowdsourcing ausgebildet. Damit wird es ermöglicht, dosisrelevante Information von vielen Geräten und Benutzern zu sammeln, diese zu clustern und daraus durch Lernalgorithmen automatisch Steuerungsinformationen, insbesondere hinsichtlich einer Dosiseinstellung, abzuleiten. Durch den Crowdsourcingaspekt lernt kann jede angeschlossene Einheit bzw. jeder Teilnehmer des Netzwerksystems und kann davon profitieren. Durch Abfrage einer zentralen Dosis-Bild-Datenbank DDB können so automatisch bessere Einstellungen für das jeweilige Gerät gefunden werden. "Bessere Einstellungen" bezieht sich insbesondere auf eine Dosisreduktion, die alleine auf Basis von einer automatischen Bildanalyse der Bilder erzielt werden kann. Die Modalitäten 10 senden die von Ihnen erfassten Bilddaten oder Bilder über das Netzwerk nw an die Dosis-Bild-Datenbank DDB. Die Dosis-Bild-Datenbank DDB speichert Bilder und zugeordnet dazu Dosismesswerte, Qualitätsmesswerte und weitere Daten und hält diese zugriffsbereit vor. Die Daten können mit unterschiedlichen Schlüsseln abgerufen werden. Die Datenbank DDB steht in Datenaustausch mit einer Steuereinheit S, die in einer Variante eine Visualisierungseinheit V umfassen kann. Die Visualisierungseinheit V dient zur Anzeige von Bildern auf einer Anzeigevorrichtung; letztere kann auch getrennt von der Visualisierungseinheit V und/oder von der Steuereinheit S bereitgestellt werden, z.B. auf einer der bildgebenden Einrichtungen 10. Damit besteht für die Anwender der Vorteil, dass er die Bilder, deren Qualität gemessen und die zu Berechnung der Dosis berücksichtigt worden sind auch sehen kann uns somit die Berechnung bzw. die Grundlagen der Berechnung auch nachvollziehen kann. Mit diesem Merkmal kann die Steuerung auf Plausibilität überprüft werden. Des Weiteren kann die Visualisierungseinheit V auch dazu dienen, die berechneten Steuergrößen mit der berechneten Dosis anzuzeigen, was wiederum einen zusätzliche Überprüfung ermöglicht und damit die Qualität insgesamt steigern vermag.

In der Dosis-Bild-Datenbank DDB ist eine Zuordnungsrelation abgelegt. Sie umfasst zu jedem Bild oder zu einer Klasse von Bildern:
- eine Dosisinformation bzw. einem Dosismesswert,
- eine Bildqualitätsinformation bzw. einen Bildqualitätsmesswert.

In Weiterbildungen der Erfindung ist es hilfreich, noch weitere Daten bereitzustellen, wie:
- die jeweilige anatomische Körperregion (Schädel, Knie, Herz etc.),
- der Status der Bilddaten (Kind, Erwachsener), und/oder
- gegebenenfalls weitere Daten, die auch automatisch aus dem DICOM Header ausgelesen und verarbeitet werden können.

Jede oder ausgewählte bildgebende Einrichtung(en) 10 umfassen vorzugsweise eine Sendeeinheit Se, die dazu bestimmt ist, die von der bildgebenden Einrichtung 10 erfassten Bilder an die Dosis-Bild-Datenbank DDB zu senden, sobald diese als Bilddatensatz vorliegt. Die Sendeeinheit Se kann nach unterschiedlichen Mustern gesteuert werden. Vorzugsweise werden automatisch alle Bilder an die Dosis-Bild-Datenbank DDB gesendet. Es ist auch möglich, die Sendeeinheit Se in einem Pooling-Modus zu betreiben und erst eine konfigurierbare Anzahl von Bilddatensätzen zu sammeln und dann als Paket an die Datenbank DDB weiterzureichen.

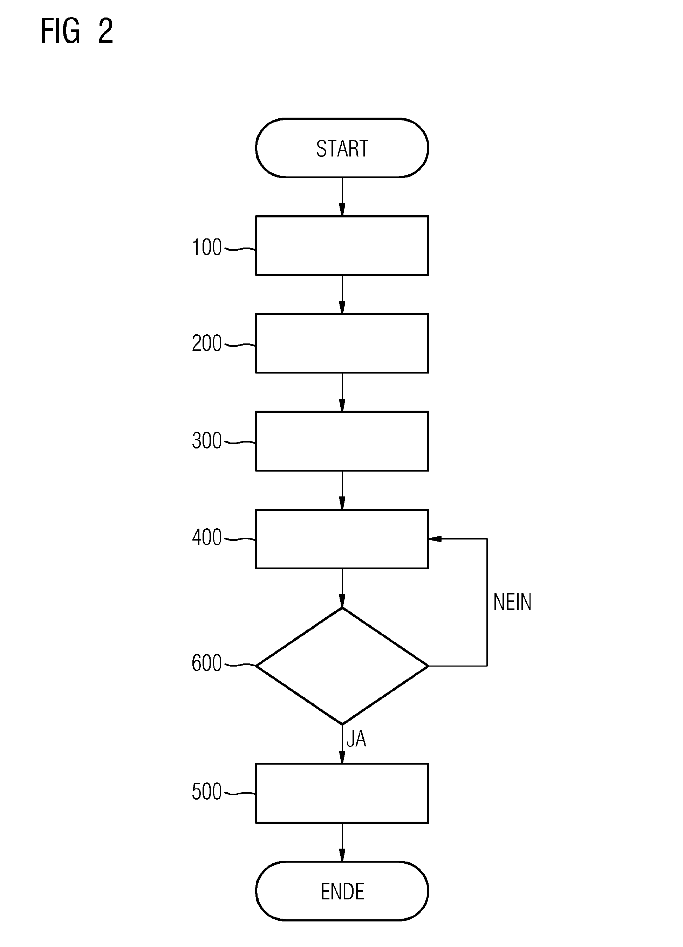

Im Folgenden sei das erfindungsgemäße Verfahren gemäß einer bevorzugten Ausführungsform unter Bezugnahme auf Figur 2 näher erläutert.

Nach dem Start des Verfahrens werden in Schritt 100 die auf der Modalität 10 akquirierten und gegebenenfalls berechneten Bilddaten an die Dosis-Bild-Datenbank DDB gesendet.

In Schritt 200 wird eine Zuordnungsrelation in der und für die Datenbank DDB erstellt. Dabei wird zu jedem Bild oder zu einer Klasse von ähnlichen Bildern weitere Datensätze zugeordnet. Diese umfassen, wie oben bereits beschrieben, zumindest eine Dosisinformation und eine Bildqualitätsinformation.

In Schritt 300 wird diese Zuordnung gespeichert.

Üblicherweise werden die Bilddaten im DICOM Format als sogenannte Studien erfasst, die jeweils eine Vielzahl von Bildern umfassen, z.B. eine Menge von Schichtbildaufnahmen für einen dreidimensionalen Datensatz. Für eine solche Studie oder für eine andere Menge von Bildern kann ein Stellvertreter als repräsentatives Bild für die Klasse bestimmt werden. Der Bildklassenrepräsentant soll die Menge von Bildern in dieser Klasse oder Gruppe kennzeichnen. Die Kriterien zur Klassenbildung sind variabel und können von Fall zu Fall angepasst werden. In einer bevorzugten Ausführungsform erfolgt die Klassenbildung in der Cluster-Einheit, die das Bezugszeichen 70 trägt. Die Cluster-Einheit 70 kann in die Datenbank eingebunden oder an sie über eine Netzwerkverbindung nw angeschlossen sein.

In Schritt 400 werden Steuergrößen für die anstehende Untersuchung erzeugt. Die Steuergröße ist in einer bevorzugten Ausführungsform eine Dosiskenngröße. Alternativ kann sie noch weitere Steuerparameter umfassen.

In Schritt 500 werden die berechneten Steuergrößen an die jeweiligen Modalitäten 10 weitergeleitet und verteilt. Dabei ist es möglich, dass gleichzeitig und sozusagen parallel für mehrere Modalitäten 10 die Steuergrößen berechnet werden. Dann sind die Steuergrößen modalitätsspezifisch und werden über eine dem Steuerdatensatz zugeordneten Identifikator an die jeweils relevante Modalität weitergeleitet.

Wie in Figur 2 dargestellt kann in einer Variante der Erfindung vorgesehen sein, dass die berechneten Steuergrößen in Schritt 600 durch ein Bestätigungssignal eines Anwenders validiert/bestätigt oder verworfen werden. Im Fall der Bestätigung fährt das Verfahren mit Schritt 500 fort. Im Fall des Verwerfens kann das Verfahren enden oder es kann erneut der Schritt 400 mit dem Erzeugen von Steuergrößen ausgeführt werden.

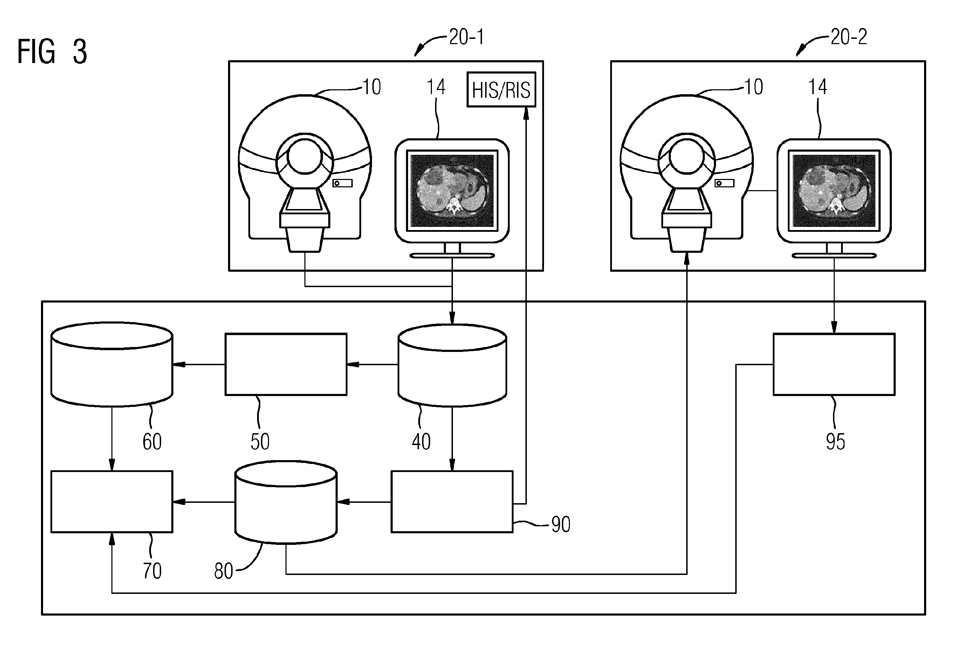

Figur 3 zeigt in einer schematischen Darstellung eine Variante des Netzwerkes mit weiteren Instanzen. Die Modalitäten 10 sind in diesem Fall Computertomographen, die in Figur 3 oben dargestellt sind. Die Modalitäten 10 stehen in Datenaustausch mit einer Workstation 14 und diese kann auch mit einem Radiologie-Informationssystem (Radiology Information System) RIS und/oder mit einem Krankenhausinformationssystem (hospital information system) HIS in Datenaustausch stehen. In Figur 3 sind nur jeweils zwei Modalitäten dargestellt; üblicherweise sind mehrere Modalitäten 10 zu einer Site 20-1, 20-2 zusammengeschlossen. Die CT-Modalität 10 (der Scanner) oder die zugeordnete Workstation 14 lädt die von ihr akquirierten Bilder automatisch in ein Cloud-basiertes Netzwerksystem, insbesondere in eine Bilddatenbank (image database) 40. Auf diese Bilddatenbank 40 greift ein Bildmerkmalsextraktor 50 (der auch als Qualitätsbestimmungseinheit bezeichnet werden kann; insofern sind diese beiden Begriffe als synonym zu versehen) zu, um einen Bildverarbeitungsalgorithmus (zur Extraktion von Bildmerkmalen) auf allen oder ausgewählten in der Bilddatenbank gespeicherten Bildern auszuführen. Die extrahierten Merkmale dienen zur Berechnung einer Bildqualität. Die Bildqualität kann insbesondere durch Algorithmen berechnet werden, die eine Abgrenzbarkeit von anatomischen Strukturen (z.B. Organen) vom Bildhintergrund (z.B. umliegendes Gewebe) - die sogenannte smoothness - kennzeichnen. Darüber hinaus kann auch ein Signal/Rauschverhältnis (S/N signal noise ratio) berechnet werden. Die extrahierten Merkmale, die die Bildqualität kennzeichnen, werden in einer Bildmerkmalsdatenbank 60 gespeichert. Zusätzlich kann auch noch Metainformation über den Patienten gespeichert werden, wie z.B. Form/Umfang, Größe, Geschlecht, Status (männlich, weiblich) etc..

Der Dosisdatenextraktor 90 (dose data extractor) greift auf das Radiologie-Informationssystem RIS und/oder auf das Krankenhausinformationssystem HIS zu, um Datensätze in Bezug auf die Erkrankung des Patienten zu erfassen. Zusätzlich können weitere anatomische oder medizinische Hintergrundinformationen ausgelesen werden, die relevant für die Dosisberechnung sind. Des Weiteren wird das der jeweiligen Aufnahme zugrundeliegende Protokoll und die Strahlendosisdaten aus einem dem Bild zugeordneten Bilddatensatz (vorzugsweise aus dem DICOM Header) ausgelesen. Diese Dosismetadaten werden in einem Dosisspeicher 80 gespeichert, der auch als Datenbank organisiert sein kann. In einer bevorzugten Ausführungsform entspricht der Dosisdatenspeicher 80 der Dosis-Bild-Datenbank DDB. Es ist jedoch auch möglich, hier unterschiedliche Instanzen vorzusehen.

Eine Cluster-Einheit 70 greift auf die in der Bildmerkmalsdatenbank 60 gespeicherten Bildmerkmale zu und klassifiziert die Bilder in Klassen (Bildklassen) oder Gruppen. Dazu verwendet sie vorzugsweise eine Multi-Class Trainingsalgorithmus, um ähnliche Fälle mit ähnlichen Bildern und ähnlichen Dosismesswerten (Einstellungen und Protokollen, settings) zu bestimmen. Diese so berechneten Trainingsergebnisse werden dann zurück in den Dosisspeicher 80 gespeichert. Zusätzlich berechnet die Cluster-Einheit 70 repräsentative Bilder und Dosiseinstellungen (dose settings) für jedes Cluster (Klasse), die dann später in einer Dosisbestimmungsphase (query phase) verwendbar sind. Damit können die für die Dosisberechnung relevanten Parameter konstant bzw. kontinuierlich verfeinert werden. Damit kann die Qualität für die Steuerung des Gerätes hinsichtlich der einzustellenden Dosis deutlich gesteigert werden.

Später, während der Dosisbestimmungsphase kann eine andere Site 20-n beispielsweise auf den Dosisspeicher 80 zugreifen und die dort hinterlegten Datensätze mittels einer Abfrageeinheit 95 (query unit) durchsuchen lassen, in dem automatisch in der Cluster-Einheit 70 nach passenden repräsentativen Bildern gesucht wird, um daraus automatisch eine optimale Einstellung mit den passenden Steuergrößen für die anstehenden Untersuchung zu berechnen. Nach der Berechnung kann dann das jeweilige Protokoll automatisch von dem Dosisspeicher 80 auf den Scanner geladen und angewendet werden. Aber das Netzwerksystem ist auch intelligent genug, um proaktiv Protokolleinstellungen als Steuergrößen zu erzeugen. Dazu kann in einer einfachen Ausführungsform der Erfindung ein Auswertealgorithmus angewendet werden, um aus den Metadaten (Krankheit des Patienten) und der angeforderten Prozedur (z.B. Schädel-CT) automatisch Steuergrößen und Dosiszielwerte zu ermitteln.

Der Anwender kann darüber hinaus das jeweilige Protokoll mit der Dosisberechnung über die Selektion der Bilder bestimmen. In diesem Fall kennzeichnet (z.B. markiert) er diejenigen Bilder oder auch repräsentativen Bilder, deren Bildqualität er als ausreichend (oder als am besten geeignet) erachtet. Daraufhin wird vom System automatisch der jeweils zugeordnete Dosisreport geöffnet (der in dem Dosisspeicher 80 abgelegt sein kann), um die Dosis zu bestimmen. Daraufhin können die jeweiligen Steuerparameter zur Bestimmung des Dosiswertes für die anstehende Untersuchung übernommen werden. Diese Auswahlmöglichkeit anhand der Bilder ist für den Anwender sehr vorteilhaft, da er sich lediglich in den Bildern orientieren muss und keine weiteren gedanklichen Umsetzungen (z.B. in Form von Zahlen oder anderen Metriken) vornehmen muss. Er kann einfach dasjenige Referenzbild (aus der Menge der in der Datenbank abgelegten Voraufnahmen) auswählen, dessen Qualität er als ausreichend erachtet. Automatisch wird zu diesem Referenzbild die jeweilig zugeordnete Dosis bestimmt und als Ziel-Strahlendosis ausgegeben.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Für einen Fachmann ist es insbesondere offensichtlich, dass die Erfindung teilweise oder vollständig in Soft- und/oder Hardware und/oder auf mehrere physikalische Produkte - dabei insbesondere auch Computerprogrammprodukte - verteilt realisiert werden kann, z.B. Modalitäts-seitig (client site-seitig) oder zentral (Dosis-Bild-Datenbank-seitig bzw. host-seitig).

### Bezugszeichenliste

- 10: bildgebende Einrichtung (Modalität)
- 20: Site, Netzwerkeinheit
- 20-1: erste Site
- 20-2: zweite Site
- HIS: Krankenhausinformationssystem (hospital information system)
- RIS: Radiologieinformationssystem (radiology information system)
- 14: Workstation

- 40: Bilddatenbank (image database)
- 50: Bildmerkmalsextraktor (image features extractor)
- 60: Bildmerkmalsdatenbank (image features database)
- 70: Cluster-Einheit (clustering unit)
- 80: Dosisspeicher (dose registry)
- 90: Dosisdatenextraktor (dose data extractor)
- 95: Abfrageeinheit (query unit)

- ap: Auswahlparameter
- S: Steuereinheit
- V: Visualisierungseinheit
- DDB: Dosis-Bild-Datenbank
- Nw: Netzwerk

- 100: Automatisches Senden aller oder ausgewählter Bilder
- 200: Erstellen einer Zuordnung oder Zuordnungsrelation
- 300: Speichern der Zuordnung
- 400: Erzeugen von Steuergrößen
- 500: Weiterleiten der erzeugten Steuergrößen
- 600: User-Validierung/Signaleingang: Wurde Bestätigungssignal empfangen?

## Patentansprüche

1. Computer-implementiertes Netzwerksystem zum Zugriff auf eine Dosis-Bild-Datenbank (DDB) für die medizinische Bildgebung unter Anwendung von ionisierender Strahlung mittels einer bildgebenden Einrichtung (10), mit:
- Einer Vielzahl von bildgebenden Einrichtungen (10), die über eine Netzwerkverbindung (nw) an die Dosis-Bild-Datenbank (DDB) angeschlossen sind, wobei die bildgebende Einrichtung (10) eine Sendeeinheit (Se) umfasst, die dazu bestimmt ist, die von der bildgebenden Einrichtung (10) erfassten Bilder an die Dosis-Bild-Datenbank (DB) zu senden,
wobei in der Dosis-Bild-Datenbank (DDB) eine Zuordnung zwischen einem Bild oder eine Klasse von Bildern, einem Dosismesswert, einer Bildqualitätsmesswert und optional noch weiteren medizinischen Metadaten abgelegt ist
- Einer Steuereinheit (S), die in Datenaustausch steht mit der Dosis-Bild-Datenbank (DDB) und die dazu dient, unter Zugriff auf die Dosis-Bild-Datenbank (DDB) automatisch Steuergrößen in Abhängigkeit von zumindest einem zu bestimmenden Auswahlparameter zu erzeugen, die über eine Netzwerkverbindung (nw) an zumindest eine bildgebende Einrichtung (10) zur Steuerung der Bildgebung weitergeleitet werden.

2. Computer-implementiertes Netzwerksystem nach Patentanspruch 1, wobei die Steuereinheit (S) zentral für alle bildgebenden Einrichtungen (10) angeordnet ist.

3. Computer-implementiertes Netzwerksystem nach einem der vorstehenden Patentansprüche, wobei die Steuereinheit (S) lokal auf der bildgebenden Einrichtung (10) angeordnet ist.

4. Computer-implementiertes Netzwerksystem nach einem der vorstehenden Patentansprüche, wobei das Netzwerksystem eine Cluster-Einheit (12) umfasst, die dazu bestimmt ist, für eine Menge von Bildern ein Referenzbild zu bestimmen, und, dass nur für das Referenzbild die Zuordnung in der Dosis-Bild-Datenbank (DDB) abgelegt wird.

5. Computer-implementiertes Netzwerksystem nach einem der vorstehenden Patentansprüche, wobei der Auswahlparameter einen oder mehrere der folgenden Parameter umfassen kann:
- Patientenidentität
- Zu untersuchender, anatomischer Bereich,
- gewünschte Bildqualität.

6. Computer-implementiertes Netzwerksystem nach einem der vorstehenden Patentansprüche, das zusätzlich eine Qualitätsbestimmungseinheit (50) umfasst, die dazu bestimmt ist, den Bildqualitätsmesswert automatisch zu bestimmen.

7. Computer-implementiertes Netzwerksystem nach einem der vorstehenden Patentansprüche, bei dem die Steuereinheit (S) weiter dazu ausgebildet ist, ein Protokoll zu bestimmen und, gegebenenfalls nach Empfang eines Bestätigungssignals hin, automatisch anzuwenden.

8. Computer-implementiertes Netzwerksystem nach einem der vorstehenden Patentansprüche, das zusätzlich eine Visualisierungseinheit umfasst, die dazu bestimmt ist, von der Steuereinheit (S) berücksichtige Referenzbilder zu visualisieren.

9. Computer-implementiertes Verfahren zum Austausch von Steuergrößen für eine bildgebende Einrichtung (10) zur Bildgebung mit ionisierender Strahlung unter Zugriff auf eine Dosis-Bild-Datenbank (DDB), mit folgenden Verfahrensschritten:
- Automatisches Senden (100) aller oder ausgewählter Bilder, die von einer oder mehreren bildgebenden Einrichtungen (10) erfasst werden, über eine Netzwerkverbindung (nw) an eine Dosis-Bild-Datenbank (DDB),
- Erstellen (200) einer Zuordnung und Speichern (300) derselben in der Dosis-Bild-Datenbank (DDB) zwischen einem Bild oder eine Klasse von Bildern, einem Dosismesswert, einer Bildqualitätsmesswert und optional noch weiteren medizinischen Metadaten
- Erzeugen (400) von Steuergrößen in Abhängigkeit von zumindest einem zu bestimmenden Auswahlparameter unter Zugriff auf die Dosis-Bild-Datenbank (DDB),
- Weiterleiten (500) der erzeugten Steuergrößen über eine Netzwerkverbindung (nw) an zumindest eine bildgebende Einrichtung (10) zur Steuerung der Bildgebung.

10. Computerprogrammprodukt, wobei das Computerprogrammprodukt ein Computerprogramm umfasst, das auf einem Datenträger oder auf einem Speicher eines Computers gespeichert ist und das von dem Computer lesbare Befehle umfasst, die zur Ausführung des Verfahrens nach dem vorstehenden Verfahrensanspruch bestimmt sind, wenn die Befehle auf dem Computer ausgeführt werden.
